# EUROPÄISCHE PATENTSCHRIFT

(11) **EP 4 256 326 B1**
(45) Veröffentlichungstag und Bekanntmachung des Hinweises auf die Patenterteilung: **02.12.2026**
(21) Anmeldenummer: 21801911.5
(22) Anmeldetag: 28.10.2021
(51) Int. Cl.: G01N 33/38, G01N 31/00

(54) **VERFAHREN ZUR QUANTITATIVEN BESTIMMUNG VON AL4C3**
METHOD FOR QUANTITATIVE DETERMINATION OF AL4C3
PROCÉDÉ DE DÉTERMINATION QUANTITATIVE DU AL4C3

(30) Priorität: 04.12.2020 EP 20211921
(43) Veröffentlichungstag der Anmeldung: 11.10.2023
(73) Patentinhaber: Refractory Intellectual Property GmbH & Co. KG, 1100 Wien (AT)
(72) Erfinder: HEID, Stefan, 8700 Leoben (AT); NILICA, Roland, 8733 Sankt Marein-Feistritz (AT); ELLERSDORFER, Markus, 8700 Leoben (AT); NIEDERMAYER, Stefan, 3430 Nitzing (AT)
(74) Vertreter: Berkenbrink, Kai-Oliver
(86) Internationale Anmeldenummer: PCT/EP2021/080052
(87) Internationale Veröffentlichungsnummer: WO 2022/117268

(56) Entgegenhaltungen:
- DE-T5- 112009 004 278
- JP-A- S62 198 757
- SIMENSEN C J: "Gas-Chromatographic Analysis of Carbides in Aluminium and Magnesium", FRESENIUS Z. ANAL. CHEM, vol. 292, 1 January 1978 (1978-01-01), pages 207 - 212, XP003031417
- FAVRE A ET AL: "Reaction Between Liquid Al (or Si) and Composite C/C Materials", HIGH TEMPERATURE CERAMIC MATRIX COMPOSITES. INTERNATIONAL CONFERENCE ON HIGH TEMPERATURE CERAMIC COMPOSITES, 12 October 2001 (2001-10-12), pages 334 - 340, XP009064005, ISBN: 9783527303205, DOI: 10.1002/3527605622.CH53
- EDTMAIER CHRISTIAN ET AL: "Microstructural characterization and quantitative analysis of the interfacial carbides in Al(Si)/diamond composites", JOURNAL OF MATERIAL SCIENCE, SPRINGER, vol. 53, no. 22, 30 July 2018 (2018-07-30), pages 15514 - 15529, XP036578234, ISSN: 0022-2461, DOI: 10.1007/S10853-018-2734-1
- SIEGENTHALER U ET AL: "Conversion of water to a counting gas for low-level tritium measurements by means of aluminium carbide", INTERNATIONAL JOURNAL OF APPLIED RADIATION AND ISOTOPS, PERGAMON PRESS, NEW YORK, NY, US, vol. 26, no. 8, 1 August 1975 (1975-08-01), pages 459 - 464, XP024704988, ISSN: 0020-708X, DOI: 10.1016/0020-708X(75)90061-7
- NANDY S K ET AL: "Hydration of coked MgO-C-Al refractories", CERAMICS INTERNATIONAL, ELSEVIER, AMSTERDAM, NL, vol. 32, no. 2, 25 April 2005 (2005-04-25), pages 163 - 172, XP024914302, ISSN: 0272-8842, DOI: 10.1016/J.CERAMINT.2005.01.013

## Beschreibung

Die Erfindung betrifft ein Verfahren zur quantitativen Bestimmung von Al₄C₃.

Al₄C₃ ist ein Aluminiumcarbid, das als Reaktionsprodukt aus Aluminium (Al) und Kohlenstoff (C) insbesondere bei hohen Temperaturen entsteht.

Al₄C₃ hat die Eigenschaft, in Gegenwart von Wasser, beispielsweise in Form von flüssigem Wasser oder von Luftfeuchtigkeit, zu Aluminiumhydroxid und Methan zu reagieren. Dies kann zu Problemen führen, wenn sich Al₄C₃ umfassende Produkte in einer feuchten Umgebung befinden.

Ein typisches Problem stellt die Anwesenheit von Al₄C₃ in feuerfesten Erzeugnissen dar. Al₄C₃ kann sich in wesentlichen Anteilen insbesondere in kohlenstoffgebundenen feuerfesten Erzeugnissen bilden, denen Aluminium als Antioxidationsmittel zugegeben wird. Während des Einsatzes dieser feuerfesten Erzeugnisse bei hohen Temperaturen bildet sich aus Kohlenstoff und Aluminium Al₄C₃. Soweit diese feuerfesten Erzeugnisse nach der Bildung von Al₄C₃ dauerhaft erhöhten Temperaturen ausgesetzt sind, ist die Anwesenheit von Al₄C₃ in dem feuerfesten Erzeugnis regelmäßig nicht problematisch. Problematisch kann Al₄C₃ jedoch beispielsweise dann werden, wenn solch gebrauchte feuerfeste Erzeugnisse nach ihrem Einsatz als rezyklierter Rohstoff für die Herstellung von neuen feuerfesten Erzeugnissen eingesetzt werden. Denn während der Herstellung oder Lagerung eines solch rezyklierten feuerfesten Rohstoffs oder eines daraus hergestellten neuen feuerfesten Erzeugnisses kann das Al₄C₃ mit Feuchtigkeit, beispielsweise aus der Umgebungsluft, hydratisieren und aufgrund der damit einhergehenden Volumenausdehnung zur Beschädigung oder auch Zerstörung des neuen feuerfesten Erzeugnisses führen.

Soweit Al₄C₃ jedoch nur in geringen Anteilen in einem feuerfesten Erzeugnis vorliegt, ist Al₄C₃ in der Regel tolerierbar. Gleichzeitig besteht das Bedürfnis, Al₄C₃ umfassende, gebrauchte feuerfeste Erzeugnisse als rezyklierten Rohstoff für die Herstellung neuer feuerfester Erzeugnisse zu verwenden. Um den durch einen rezyklierten Rohstoff in ein neues feuerfestes Erzeugnis eingetragenen Anteil an Al₄C₃ bestimmen zu können, ist es daher notwendig, den Anteil an Al₄C₃ in dem gebrauchten feuerfesten Erzeugnis, das als rezyklierter Rohstoff eingesetzt werden soll, quantitativ bestimmen zu können.

Simensen C J ("Gas-Chromatographic Analysis of Carbides in Aluminium and Magnesium", Fresenius Z. Anal. Chem., Bd. 292. 1. Januar 1978, Seiten 207-212) offenbart eine gas-chromatographische Methode zur quantitativen Bestimmung von Al₄C₃ in Aluminium, bei welcher Aluminium in einer in einem evakuierten Glaskolben befindlichen Lösung aus Natriumhydroxid gelöst, das dabei entstehende Methan gemessen und auf Basis dieser Messung der Gehalt an Al₄C₃ im Aluminium bestimmt wird. Edtmaier C et al ("Microstructural characterization and quantitative analysis of interfacial carbides in Al(Si)/diamond composites", Journal of Material Science, Bd. 53(22), 30. Juli 2018, Seiten 15514-15529) offenbaren eine gas-chromatographische Methode zur quantitativen Bestimmung von Al₄C₃ in einem Al(Si)/Diamant-Kompositwerkstoff, bei welcher der Kompositwerkstoff in einer in einem gasdicht verschlossenen Gefäß befindlichen Lösung aus Natriumhydroxid gelöst, das dabei entstehende Methan gemessen und auf Basis dieser Messung der Gehalt an Al₄C₃ im Kompositwerkstoff bestimmt wird.

Der Erfindung liegt die Aufgabe zugrunde, ein Verfahren zur quantitativen Bestimmung von Al₄C₃ zur Verfügung zu stellen. Insbesondere soll ein solches Verfahren zur zuverlässigen quantitativen Bestimmung von Al₄C₃ zur Verfügung gestellt werden. Insbesondere soll ein solches Verfahren zur zuverlässigen, einfachen und sicheren quantitativen Bestimmung von Al₄C₃ zur Verfügung gestellt werden.

Zur Lösung der Aufgabe wird erfindungsgemäß ein Verfahren zur quantitativen Bestimmung von Al₄C₃ zur Verfügung gestellt, das die folgenden Schritte umfasst:
Zur Verfügungstellung eines gasdicht verschließbaren Raumes;
zur Verfügungstellung einer Al₄C₃ umfassenden Substanz, wobei die Al₄C₃ umfassende Substanz vorzugsweise in Form eines feuerfesten Erzeugnisses zur Verfügung gestellt wird;
zur Verfügungstellung wenigstens einer wässrigen Flüssigkeit, die unter Bildung wenigstens eines Gases mit Al₄C₃ reagiert;
anordnen der Al₄C₃ umfassenden Substanz und der wenigstens einen wässrigen Flüssigkeit in dem Raum;
gasdichtes Verschließen des Raumes;
reagieren lassen des Al₄C₃ der Al₄C₃ umfassenden Substanz und der wenigstens einen wässrigen Flüssigkeit in dem Raum unter Bildung des wenigstens einen Gases;
quantitative Bestimmung des gebildeten wenigstens einen Gases;
quantitative Bestimmung von Al₄C₃ in der Al₄C₃ umfassenden Substanz auf Basis der quantitativen Bestimmung des gebildeten wenigstens einen Gases; wobei
der Raum während der Reaktion des Al₄C₃ der Al₄C₃ umfassenden Substanz mit der wenigstens einen wässrigen Flüssigkeit in dem Raum unter Überdruck im Bereich von 2-6 bar steht.

Die Erfindung beruht auf der überraschenden Erkenntnis, dass durch ein solches Verfahren ein besonders zuverlässiges, einfaches und sicheres Verfahren zur quantitativen Bestimmung von Al₄C₃ zur Verfügung gestellt werden kann. Dabei beruht die Erfindung insbesondere auch auf der überraschenden Erkenntnis, dass das Verfahren insbesondere auch aufgrund der Verwendung der wässrigen Flüssigkeit sowie des gasdichten Verschließens des Raumes besonders zuverlässig, einfach und sicher durchführbar ist.

Gemäß dem erfindungsgemäßen Verfahren wird der Anteil an Al₄C₃ in der Al₄C₃ umfassenden Substanz nicht unmittelbar bestimmt. Vielmehr wird das durch die Reaktion des Al₄C₃ der Al₄C₃ umfassenden Substanz und der wenigstens einen wässrigen Flüssigkeit in dem Raum gebildete, wenigstens eine Gas quantitativ bestimmt und der Anteil an Al₄C₃ in der Al₄C₃ umfassenden Substanz mittelbar auf Basis der quantitativen Bestimmung des gebildeten wenigstens einen Gases quantitativ bestimmt.

Bevorzugt werden bei der Durchführung des erfindungsgemäßen Verfahrens zunächst die Al₄C₃ umfassende Substanz und die wenigstens eine wässrige Flüssigkeit in dem Raum angeordnet und der Raum anschließend gasdicht verschlossen. Anschließend lässt man das Al₄C₃ der Al₄C₃ umfassenden Substanz und die wenigstens eine wässrige Flüssigkeit in dem Raum unter Bildung des wenigstens einen Gases miteinander reagieren. Bevorzugt bleibt der Raum während dieser Reaktion gasdicht verschlossen. Insbesondere bleibt der Raum bevorzugt gasdicht verschlossen, bis das Al₄C₃ der Al₄C₃ umfassenden Substanz und die wenigstens eine wässrige Flüssigkeit in dem Raum unter Bildung des wenigstens einen Gases vollständig miteinander reagiert haben. Anschließend, also nach der Reaktion des Al₄C₃ der Al₄C₃ umfassenden Substanz und der wenigstens einen wässrigen Flüssigkeit in dem Raum unter Bildung des wenigstens einen Gases, wird das gebildete wenigstens eine Gas quantitativ bestimmt. Bevorzugt wird das gebildete wenigstens eine Gas quantitativ bestimmt, nachdem die Reaktion abgeschlossen ist, also nachdem das Al₄C₃ der Al₄C₃ umfassenden Substanz und die wenigstens eine wässrigen Flüssigkeit in dem Raum unter Bildung des wenigstens einen Gases vollständig miteinander reagiert haben. Je nach angewandter Methode zur quantitativen Bestimmung des gebildeten wenigstens einen Gases kann der Raum nach der Reaktion des Al₄C₃ der Al₄C₃ umfassenden Substanz und der wenigstens einen wässrigen Flüssigkeit in dem Raum unter Bildung des wenigstens einen Gases zur quantitativen Bestimmung des gebildeten wenigstens einen Gases entweder geöffnet werden oder gasdicht verschlossen bleiben. Anschließend, also nach der quantitativen Bestimmung des gebildeten wenigstens einen Gases, wird das Al₄C₃ in der Al₄C₃ umfassenden Substanz auf Basis der quantitativen Bestimmung des gebildeten wenigstens einen Gases quantitativ bestimmt.

Nach einer besonders bevorzugten Ausführungsform handelt es sich bei dem wenigstens einen Gas, das sich bei der Reaktion des Al₄C₃ der Al₄C₃ umfassenden Substanz und der wenigstens einen wässrigen Flüssigkeit in dem Raum bildet, um Methan. Bekanntermaßen reagiert Al₄C₃ nach der folgenden Reaktionsgleichung (I) mit Wasser zu Aluminiumhydroxid und Methan:

Al₄C₃ + 12 H₂O → 4 Al(OH)₃ + 3 CH₄ ^{↑} (I)

Bei der Durchführung des erfindungsgemäßen Verfahrens reagiert das Al₄C₃ der Al₄C₃ umfassenden Substanz mit der wenigstens einen wässrigen Flüssigkeit in dem Raum entsprechend der vorstehenden Reaktionsgleichung (I) zu Aluminiumhydroxid und gasförmigem Methan. Auf Basis der quantitativen Bestimmung des dabei gebildeten gasförmigen Methans lässt sich daher der Anteil an Al₄C₃ in der Al₄C₃ umfassenden Substanz quantitativ sehr genau bestimmen.

Zur quantitativen Bestimmung des gebildeten wenigstens einen Gases, also bevorzugt des Methans, kann grundsätzlich auf die aus dem Stand der Technik bekannten Methoden zur quantitativen Bestimmung von Gasen, insbesondere zur quantitativen Bestimmung von Methan, zurückgegriffen werden.

Beispielsweise kann die quantitative Bestimmung des wenigstens einen Gases mittels einer gasanalytischen Bestimmung durchgeführt werden. Hierbei wird das wenigstens eine Gas mittels der bekannten gasanalytischen Messmethoden quantitativ bestimmt. Beispielsweise kann auf Basis der Messung der Konzentration des wenigstens einen Gases in dem Raum das wenigstens eine Gas quantitativ bestimmt werden. Hierzu kann beispielsweise die Konzentration des in dem Raum gebildeten wenigstens einen Gases in einem Gasvolumen gemessen und ferner das Gasvolumen gemessen und auf Basis dieser Messungen das wenigstens eine Gas quantitativ bestimmt werden. Beispielsweise kann das in dem Raum gebildete wenigstens eine Gas in ein Gasvolumen eingeleitet und die Konzentration des wenigstens einen Gases in diesem Gasvolumen gemessen und ferner das Gasvolumen gemessen und auf Basis dieser Messungen das wenigstens eine Gas quantitativ bestimmt werden. Die Konzentration des wenigstens einen Gases kann bevorzugt mittels eines Gassensors gemessen werden. Bevorzugt handelt es sich bei dem Gassensor um einen infrarotoptischen Gassensor. Durch einen solch infrarotoptischen Gassensor (insbesondere einen NDIR), kann die Konzentration des wenigstens einen Gases bestimmt werden, indem die optische Transmission des Gases in einem für das Gas charakteristischen Spektralbereich gemessen wird. Hieraus kann mittels des Lambert-Beer'schen Gesetzes die Konzentration des wenigstens einen Gases bestimmt werden. Das Gasvolumen kann beispielsweise durch einen Durchflussmesser bestimmt werden. Eine solch gasanalytische quantitative Bestimmung wird unten im Ausführungsbeispiel näher erläutert.

Beispielsweise kann die quantitative Bestimmung des wenigstens einen Gases alternativ auf Basis einer Druckmessung durchgeführt werden. Hierbei wird der durch die Reaktion des Al₄C₃ der Al₄C₃ umfassenden Substanz mit der wenigstens einen wässrigen Flüssigkeit in dem Raum entstehende Druck in dem Raum gemessen und auf Basis dieser Messung das wenigstens eine Gas quantitativ bestimmt. Der Druck kann bevorzugt mittels eines Drucksensors bestimmt werden. Eine solch quantitative Bestimmung des wenigstens einen Gases mittels Druckmessung wird ebenfalls unten im Ausführungsbeispiel näher erläutert.

Auf Basis der quantitativen Bestimmung des gebildeten wenigstens einen Gases kann anschließend die quantitative Bestimmung von Al₄C₃ in der Al₄C₃ umfassenden Substanz erfolgen. Hierbei kann auf Basis der Kenntnis der Reaktionsgleichung, nach welcher sich das wenigstens eine Gas bei der Reaktion des Al₄C₃ der Al₄C₃ umfassenden Substanz und der wenigstens einen wässrigen Flüssigkeit in dem Raum bildet, die Menge an Al₄C₃ in der Al₄C₃ umfassenden Substanz quantitativ bestimmt beziehungsweise berechnet werden, insbesondere mittels einer stöchiometrischen Berechnung.

Soweit beispielweise gemäß der oben angegebenen Reaktionsgleichung (I) das Al₄C₃ der Al₄C₃ umfassenden Substanz mit der wenigstens einen wässrigen Flüssigkeit in dem Raum zu einem Gas in Form Methan reagiert, lässt sich durch die quantitative Bestimmung des gebildeten Methans der Anteil an Al₄C₃ durch eine stöchiometrische Berechnung einfach quantitativ bestimmen. Denn gemäß der Reaktionsgleichung (I) reagiert jeweils ein Mol Al₄C₃ in Gegenwart von Wasser zu 3 Mol gasförmigem Methan. Entsprechend lässt sich durch die quantitative Bestimmung des gebildeten gasförmigen Methans die Menge von Al₄C₃ quantitativ bestimmen. Eine solch quantitative Bestimmung von Al₄C₃ wird unten im Ausführungsbeispiel näher erläutert.

Überraschend hat sich im Rahmen der Erfindung herausgestellt, dass das erfindungsgemäße Verfahren besonders einfach, zuverlässig und sicher durchführbar ist, soweit die Al₄C₃ umfassende Substanz mit einer wässrigen Flüssigkeit reagieren gelassen wird. Unter einer wässrigen Flüssigkeit wird im Sinne der Erfindung eine Wasser umfassende Flüssigkeit verstanden, insbesondere eine auf Wasser basierende Flüssigkeit. Nach einer besonders bevorzugten Ausführungsform liegt die wässrige Flüssigkeit in Form von Wasser vor. Nach einer besonders bevorzugten Ausführungsform ist die wässrige Flüssigkeit, insbesondere eine auf Wasser basierende Flüssigkeit, pH-neutral oder zumindest im Wesentlichen pH-neutral. Insoweit weist die wässrige Flüssigkeit bevorzugt einen pH-Wert im Bereich von 6 bis 8 und besonders bevorzugt einen pH-Wert von 7 auf.

Ein wesentlicher Vorteil einer solch wässrigen Flüssigkeit besteht darin, dass Al₄C₃ in Gegenwart einer solchen wässrigen Flüssigkeit gemäß der vorstehenden Reaktionsgleichung (I) zu Methan reagiert und über die quantitative Bestimmung des dabei gebildeten Methans der Anteil an Al₄C₃ quantitativ bestimmbar ist. Hierdurch ergibt sich eine besonders zuverlässige und einfache quantitative Bestimmung von Al₄C₃.

Ein weiterer wesentlicher Vorteil einer solch wässrigen Flüssigkeit besteht darin, dass eine solch wässrige Flüssigkeit, insbesondere Wasser oder eine pH-neutrale wässrige Flüssigkeit, besonders einfach zu handhaben ist, da eine solche Flüssigkeit das Bedienpersonal bei der Durchführung des erfindungsgemäßen Verfahrens nicht gefährdet und auch keinen aggressiven Einfluss auf die Vorrichtung zur Durchführung des erfindungsgemäßen Verfahrens ausübt.

Ein weiterer Vorteil einer solch wässrigen Flüssigkeit besteht darin, dass eine solch wässrige Flüssigkeit unter ökologischen Aspekten vorteilhaft ist.

Ein weiterer Vorteil einer solch wässrigen Flüssigkeit besteht darin, dass eine solch wässrige Flüssigkeit unter wirtschaftlichen Aspekten vorteilhaft ist, da sie kostengünstig zur Verfügung gestellt werden kann.

Nach einer bevorzugten Ausführungsform ist vorgesehen, dass die Al₄C₃ umfassende Substanz und die wenigstens eine wässrige Flüssigkeit miteinander gemischt werden. Ein solches Mischen der Al₄C₃ umfassenden Substanz mit der wenigstens einen wässrigen Flüssigkeit hat insbesondere den Vorteil, dass ein inniger Kontakt des Al₄C₃ der Al₄C₃ umfassenden Substanz mit der wenigstens einen wässrigen Flüssigkeit erreicht werden kann, so dass das Al₄C₃ vollständig mit der Flüssigkeit reagieren kann. Insbesondere kann hierdurch erreicht werden, dass das Al₄C₃ vollständig mit der wenigstens einen wässrigen Flüssigkeit unter Bildung des wenigstens einen Gases reagiert, so dass eine besonders zuverlässig quantitative Bestimmung von Al₄C₃ vorgenommen werden kann. Nach einer bevorzugten Ausführungsform werden die Al₄C₃ umfassende Substanz und die wenigstens eine wässrige Flüssigkeit miteinander gemischt, insbesondere fortwährend miteinander gemischt, während das Al₄C₃ der Al₄C₃ umfassenden Substanz und die wenigstens eine wässrige Flüssigkeit in dem Raum unter Bildung des wenigstens einen Gases miteinander reagieren gelassen werden. Dies kann beispielsweise dadurch bewerkstelligt werden, dass in dem Raum eine Mischvorrichtung vorgesehen ist, durch die die Al₄C₃ umfassende Substanz und die wenigstens eine wässrige Flüssigkeit miteinander vermischt werden können. Beispielsweise kann in dem Raum eine Mischvorrichtung in Form eines Rührers vorgesehen sein.

Für die Durchführung des erfindungsgemäßen Verfahrens wird ein gasdicht verschließbarer Raum zur Verfügung gestellt, der bei der Durchführung des erfindungsgemäßen Verfahrens gasdicht verschlossen ist. Insbesondere ist vorgesehen, dass der Raum gasdicht verschlossen ist, während das Al₄C₃ der Al₄C₃ umfassenden Substanz und die wenigstens eine wässrige Flüssigkeit in dem Raum unter Bildung des wenigstens einen Gases miteinander reagieren. Insoweit kann nach einer bevorzugten Ausführungsform vorgesehen sein, dass die für die Durchführung des erfindungsgemäßen Verfahrens zur Verfügung gestellte Al₄C₃ umfassende Substanz und die wenigstens eine wässrige Flüssigkeit zunächst in dem Raum angeordnet werden und der Raum anschließend gasdicht verschlossen wird. Anschließend lässt man das Al₄C₃ der Al₄C₃ umfassenden Substanz und die wenigstens eine wässrige Flüssigkeit in dem Raum unter Bildung des wenigstens einen Gases miteinander reagieren, während der Raum gasdicht verschlossen ist.

Ein solch gasdichtes Verschließen des Raumes während dieser Reaktion hat zahlreiche wesentliche Vorteile. Ein Vorteil besteht darin, dass das während der Reaktion des Al₄C₃ der Al₄C₃ umfassenden Substanz mit der wenigstens einen wässrigen Flüssigkeit in dem Raum gebildete wenigstens eine Gas vollständig in dem Raum aufgefangen werden kann. Dies ermöglicht eine besonders einfache und zuverlässige quantitative Bestimmung des gebildeten wenigstens einen Gases, da das gebildete wenigstens eine Gas erst nach vollständiger Reaktion quantitativ bestimmt werden kann. Hierdurch ist eine andauernde quantitative Bestimmung des wenigstens einen Gases während der gesamten Reaktionszeit nicht notwendig. Ein weiterer wesentlicher Vorteil eines solchen, während der Reaktion gasdicht verschlossenen Raumes besteht darin, dass gasförmige Anteile der wässrigen Flüssigkeit, die sich während der Reaktion des Al₄C₃ der Al₄C₃ umfassenden Substanz mit der wenigstens einen wässrigen Flüssigkeit in dem Raum bilden können, während dieser Reaktion nicht aus dem Raum entweichen können. Hierdurch kann sichergestellt werden, dass während der gesamten Reaktion eine ausreichende Menge an wässriger Flüssigkeit vorhanden ist, so dass die Reaktion aufgrund eines Mangels an wässriger Flüssigkeit nicht gehemmt wird oder sogar vollständig zum Erliegen kommt.

Bei der für das erfindungsgemäße Verfahren zur Verfügung gestellten, Al₄C₃ umfassenden Substanz kann es sich grundsätzlich um eine beliebige Substanz handeln, die Al₄C₃ umfasst. Die Al₄C₃ umfassende Substanz ist bevorzugt kohlenstoffgebunden. Nach einer besonders bevorzugten Ausführungsform wird die Al₄C₃ umfassende Substanz in Form eines feuerfesten Erzeugnisses zur Verfügung gestellt. Wie oben ausgeführt, ist insbesondere die quantitative Bestimmung des Anteils an Al₄C₃ in feuerfesten Erzeugnissen von Bedeutung, beispielsweise soweit diese als rezyklierter Rohstoff für die Herstellung von neuen feuerfesten Erzeugnissen verwendet werden sollen. Durch das erfindungsgemäße Verfahren steht insoweit ein Verfahren zur Verfügung, durch welches in solch feuerfesten Erzeugnissen der Anteil an Al₄C₃ besonders einfach, zuverlässig und sicher bestimmbar ist. Nach einer bevorzugten Ausführungsform handelt es sich bei dem feuerfesten Erzeugnis um ein kohlenstoffgebundenes feuerfestes Erzeugnis, besonders bevorzugt ein gebrauchtes kohlenstoffgebundenes feuerfestes Erzeugnis.

Unter dem Begriff "kohlenstoffgebunden" ist bevorzugt zu verstehen, dass die Al₄C₃ umfassende Substanz, bevorzugt die Al₄C₃ umfassende Substanz in Form eines feuerfesten Erzeugnisses, über eine Kohlenstoffbindung gebunden ist. Diese Kohlenstoffbindung kann ausgebildet werden, indem bei der Herstellung der Substanz ein kohlenstoffhaltiges Bindemittel zugesetzt wird. Bei dem kohlenstoffhaltigen Bindemittel kann es sich beispielsweise um Pech oder um ein synthetisches Harz, vorzugsweise ein Phenolharz, handeln. Unter einem "Phenolharz" ist ein synthetisches Harz gebildet aus einem Phenol oder einem Phenolderivat und einem Aldehyd zu verstehen.

"Gebraucht" in diesem Sinne bedeutet, dass das Erzeugnis bereits für seinen bestimmungsgemäßen Zweck eingesetzt worden ist. Bei einem solch kohlenstoffgebundenen feuerfesten Erzeugnis kann es sich besonders bevorzugt um einen Magnesiakohlenstoffstein handeln, insbesondere einen gebrauchten Magnesiakohlenstoffstein. Bekanntermaßen handelt es sich bei Magnesiakohlenstoffsteinen um feuerfeste Erzeugnisse, die hauptsächlich aus Kohlenstoff (C) und Magnesia (MgO) bestehen. Solche Steine werden auch als MgO-C-Steine bezeichnet. Bei einem solchen Magnesiakohlenstoffstein ist die Magnesia vorzugsweise über eine Kohlenstoffbindung miteinander verbunden. Bevorzugt umfasst der für die Durchführung des erfindungsgemäßen Verfahrens zur Verfügung gestellte, insbesondere gebrauchte Magnesiakohlenstoffstein einen Anteil an Kohlenstoff von 5-30 Masse-%, einen Anteil an Magnesia von 70-95 Masse-% und einen Anteil an Al₄C₃ von 0-3 Masse-%, jeweils bezogen auf die Gesamtmasse des Magnesiakohlenstoffsteins. Daneben umfasst der Magnesiakohlenstoffstein gegebenenfalls Anteile an Metallen (insbesondere Silizium und Aluminium) und Nitriden, insbesondere Aluminiumnitriden.

Das erfindungsgemäße Verfahren erweist sich zur quantitativen Bestimmung von Al₄C₃ in solchen gebrauchten Magnesiakohlenstoffsteinen insbesondere auch unter ökologischen und wirtschaftlichen Gesichtspunkten als besonders vorteilhaft, da bislang kein zuverlässiges, einfaches und sicheres Verfahren hierfür zur Verfügung stand. Dies hatte zur Ursache, dass gebrauchte Magnesiakohlenstoffsteine aufgrund eines nicht quantitativ bestimmbaren Anteils an Al₄C₃ vielfach nicht als rezyklierter Rohrstoff wiederverwendet werden konnte.

Nach einer bevorzugten Ausführungsform ist vorgesehen, dass die Al₄C₃ umfassende Substanz als Schüttgut zur Verfügung gestellt wird. Als Schüttgut in diesem Sinne wird ein schüttfähiges Gut aus Partikeln beziehungsweise Körnern bezeichnet. Bevorzugt weisen die Körner des Schüttgutes eine Korngröße unter 10 mm, noch bevorzugter unter 5 mm auf.

Ein besonderer Vorteil einer solchen als Schüttgut zur Verfügung gestellten, Al₄C₃ umfassenden Substanz liegt insbesondere darin, dass das Al₄C₃ einer solchen als Schüttgut zur Verfügung gestellten, Al₄C₃ umfassenden Substanz vollständig mit der wässrigen Flüssigkeit reagieren kann, so dass eine besonders zuverlässige quantitative Bestimmung des Anteils an Al₄C₃ ermöglicht ist.

Nach einer besonders bevorzugten Ausführungsform ist vorgesehen, dass der Raum während der Reaktion des Al₄C₃ der Al₄C₃ umfassenden Substanz mit der wenigstens einen wässrigen Flüssigkeit in dem Raum mit Temperatur beaufschlagt wird. Insbesondere kann vorgesehen sein, den Raum mit Temperatur über Raumtemperatur, also über 20° Celsius mit Temperatur zu beaufschlagen. Bevorzugt ist vorgesehen, den Raum mit Temperatur über 100° Celsius und besonders bevorzugt mit Temperatur im Bereich von 100° - 200° Celsius zu beaufschlagen. Grundsätzlich kann der Raum durch beliebige aus dem Stand der Technik bekannte Mittel zur Beaufschlagung eines Raumes mit Temperatur mit Temperatur beaufschlagt werden. Bevorzugt wird der Raum mittels einer elektrischen Heizvorrichtung mit Temperatur beaufschlagt.

Erfindungsgemäß hat sich herausgestellt, dass die Beaufschlagung des Raumes mit Temperatur mehrere Vorteile hat. Ein Vorteil besteht darin, dass die Reaktion des Al₄C₃ der Al₄C₃ umfassenden Substanz mit der wenigstens einen wässrigen Flüssigkeit beschleunigt werden kann. Hierdurch kann das erfindungsgemäße Verfahren besonders schnell und effizient durchgeführt werden. Ein weiterer besonderer Vorteil der Beaufschlagung des Raumes mit Temperatur liegt jedoch insbesondere auch darin, dass durch die Beaufschlagung des Raumes mit Temperatur in den Raum eine solch definierte Atmosphäre einstellbar ist, dass sich eines oder mehrere Gase des wenigstens einen Gases, die sich während der Reaktion des Al₄C₃ der Al₄C₃ umfassenden Substanz mit der wenigstens einen wässrigen Flüssigkeit in dem Raum bilden, in der Gasphase befinden, was eine besonders einfache Durchführung des erfindungsgemäßen Verfahrens ermöglicht. Denn hierdurch kann durch quantitative Bestimmung dieser gebildeten Gase, die sich bei der über die Temperatur eingestellten Atmosphäre im Raum in der Gasphase befinden, der Anteil des Al₄C₃ der Al₄C₃ umfassenden Substanz auf Basis dieser quantitativen Bestimmung des gebildeten wenigstens einen Gases besonders einfach quantitativ bestimmt werden.

Welche Gase sich bei einer eingestellten Atmosphäre im Raum in der Gasphase befinden, ist durch die bekannten Gasgleichungen bestimmbar und einstellbar.

Es ist vorgesehen, dass der Raum während der Reaktion des Al₄C₃ der Al₄C₃ umfassenden Substanz mit der wenigstens einen wässrigen Flüssigkeit in dem Raum unter Überdruck steht. Überdruck bezeichnet Druck über Atmosphärendruck, also einen Druck über 1 bar. Erfindungsgemäß herrscht in dem Raum während der Reaktion ein Überdruck im Bereich von 2-6 bar, bevorzugt von 3-5 bar.

Ein solcher Überdruck kann sich während der Reaktion des Al₄C₃ der Al₄C₃ umfassenden Substanz mit der wenigstens einen wässrigen Flüssigkeit in dem Raum einstellen, während dieser gasdicht abgeschlossen ist und insbesondere mit Temperatur beaufschlagt wird. Insofern sind keine zusätzlichen technischen Maßnahmen notwendig, um den Raum entsprechend mit Druck zu beaufschlagen.

Erfindungsgemäß hat sich herausgestellt, dass ein solcher Überdruck in dem Raum während der Reaktion mit zahlreichen Vorteilen verbunden ist. Zum einen wurde insoweit überraschend festgestellt, dass die Reaktion schneller abläuft, so dass die Reaktionszeit verkürzt und das Verfahren damit besonders schnell und effektiv durchgeführt werden kann. Zum Weiteren ist jedoch insbesondere auch vorteilhaft, dass, wie oben ausgeführt, durch die Beaufschlagung des Raumes mit Temperatur und Überdruck eine solch definierte Atmosphäre in dem Raum einstellbar ist, dass sich definierte Gase in der Gasphase befinden, was, wie oben ausgeführt, eine besonders einfache quantitative Bestimmung des gebildeten wenigstens einen Gases und auf dieser Grundlage auch eine besonders einfache quantitative Bestimmung von Al₄C₃ in der Al₄C₃ umfassenden Substanz ermöglicht ist.

Nach einer besonders bevorzugten Ausführungsform wird der Raum durch einen Autoklav zur Verfügung gestellt. Bekanntermaßen handelt es sich bei einem Autoklav um eine Vorrichtung, die einen gasdicht verschließbaren Raum umfasst, in dem Substanzen bei Überdruck mit Temperatur beaufschlagbar sind. Das erfindungsgemäße Verfahren lässt sich daher besonders vorteilhaft in einem Autoklav durchführen, da durch einen Autoklav nicht nur ein gasdicht verschließbarer Raum zur Verfügung gestellt wird, sondern dieser Raum zudem auch mit Temperatur und Überdruck beaufschlagbar ist.

Ein weiterer besonderer Vorteil der Verwendung eines Autoklavs zur Durchführung des erfindungsgemäßen Verfahrens besteht insbesondere auch darin, dass insoweit zur Durchführung des erfindungsgemäßen Verfahrens auf eine Vorrichtung gemäß dem Stand der Technik zurückgegriffen werden kann, um das erfindungsgemäße Verfahren auszuführen. Das erfindungsgemäße Verfahren ist unter zur Hilfenahme eines Autoklavs damit besonders einfach durchführbar.

Zur Durchführung des erfindungsgemäßen Verfahrens kann eine Vorrichtung vorgesehen sein, umfassend:
Einen gasdicht verschließbaren Raum;
Mittel zur quantitativen Bestimmung von in dem Raum gebildeten Gas.

Der gasdicht verschließbare Raum kann bevorzugt, wie oben ausgeführt, durch einen Autoklav zur Verfügung gestellt werden.

Die Mittel zur quantitativen Bestimmung von in dem Raum gebildeten Gas umfassen bevorzugt wenigstens eines der folgenden Mittel: Infrarotoptischer Gassensor oder Drucksensor.

Weitere Merkmale der Erfindung ergeben sich aus den Ansprüchen, den Figuren sowie der zugehörigen Figurenbeschreibung.

Sämtliche Merkmale der Erfindung können, einzeln oder in Kombination, im Rahmen der Ansprüche beliebig miteinander kombiniert sein.

Ausführungsbeispiele der Erfindung werden anhand der beigefügten Figur nachfolgend näher beschrieben.

Dabei zeigt
- Figur 1: ein stark schematisiertes Ausführungsbeispiel einer Vorrichtung zur Durchführung des erfindungsgemäßen Verfahrens;
- Figur 2: Messergebnisse für die Bestimmung der Methankonzentration und der Wasserstoffkonzentration bei der Durchführung des Ausführungsbeispiels des erfindungsgemäßen Verfahrens; und
- Figur 3: Messergebnisse für die Druckmessung bei der Durchführung des Ausführungsbeispiels des erfindungsgemäßen Verfahrens,

### Beispiel für eine geeignete Vorrichtung

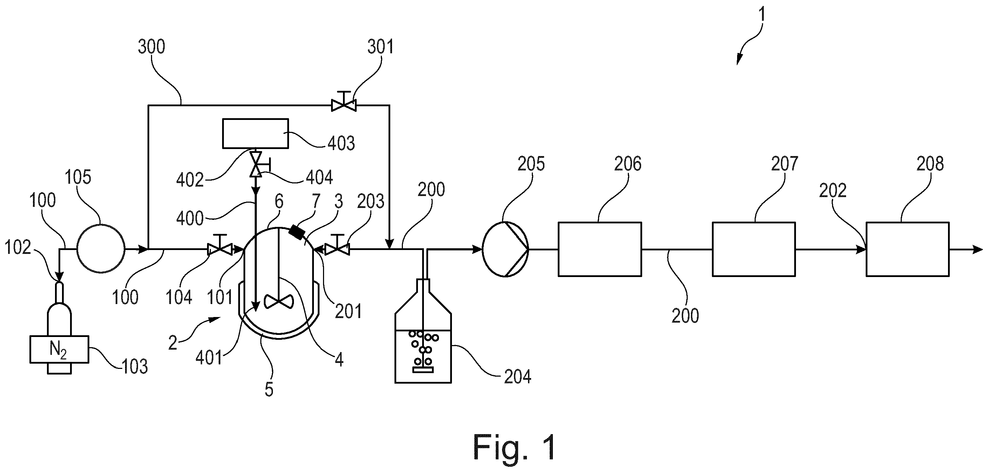

In seiner Gesamtheit ist die Vorrichtung in Figur 1 mit dem Bezugszeichen 1 gekennzeichnet.

Die Vorrichtung 1 umfasst einen Autoklav 2, der einen gasdicht verschließbaren Raum 3 umfasst. Der Autoklav 2 umfasst ferner einen Rührer 4, durch den in dem Raum 3 befindliche Substanzen gerührt und gemischt werden können, und eine elektrische Heizvorrichtung 5 zur Temperaturbeaufschlagung des Raums 3. Der Autoklav 2 weist einen Deckel 6 auf, über den der Raum 3 gasdicht verschließbar ist. Der Autoklav 2 weist ferner einen Drucksensor 7 zur Messung des Drucks im Raum 3 auf.

Durch die Wandung des Autoklavs 2 ist eine erste Gasleitung 100 geführt, die sich von einem ersten Ende 101, an dem die erste Gasleitung 100 in den Raum 3 mündet, zu einem zweiten Ende 102 erstreckt. An ihrem zweiten Ende 102 ist die erste Gasleitung an einen Stickstoff enthaltenden Stickstofftank 103 angeschlossen. Durch die erste Gasleitung 100 ist ein erster Gasleitungsweg definiert, durch den Gas entlang der ersten Gasleitung 100 vom zweiten Ende 102 zum ersten Ende 101 leitbar ist. Die erste Gasleitung 100 ist über ein Ventil 104 absperrbar.

Durch die Wandung des Autoklavs 2 ist eine zweite Gasleitung 200 geführt, die sich von einem ersten Ende 201, an dem die zweite Gasleitung 200 in den Raum 3 mündet, zu einem zweiten Ende 202 erstreckt. Durch die zweite Gasleitung 200 ist ein zweiter Gasleitungsweg definiert, durch den Gas entlang der zweiten Gasleitung 200 vom ersten Ende 201 zum zweiten Ende 202 leitbar ist. Entlang der zweiten Gasleitung 200 sind in Strömungsrichtung des zweiten Gasleitungsweges vom ersten Ende 201 zum zweiten Ende 202 die folgenden Komponenten angeordnet: Ein Ventil 203, eine strömungstechnisch dahinter angeordnete Gaswaschflasche 204, eine strömungstechnisch dahinter angeordnete Gasaufbereitungspumpe 205, ein strömungstechnisch dahinter angeordneter Gassensor 206 und ein strömungstechnisch dahinter angeordneter Leitfähigkeitsdetektor 207.

Schließich mündet die zweite Gasleitung 200 an dem strömungstechnisch hinter dem Leitfähigkeitsdetektor 207 angeordneten zweiten Ende 202 in einen Gasabzug 208. Durch das Ventil 203 ist die zweite Gasleitung 200 absperrbar. Die Gaswaschflasche 204 umfasst ein Bad aus 10%-iger Schwefelsäure, durch den der zweite Gasleitungsweg geführt ist. Durch die Gasaufbereitungspumpe 205 ist ein entlang des zweiten Gasleitungsweges geleitetes Gas auf 5°C kühlbar. Zur Steuerung des Gasdurchflusses entlang des zweiten Gasleitungsweges stromabwärts der Gasaufbereitungspumpe 205 kann der Gasvolumenstrom durch die Gasaufbereitungspumpe 205 eingestellt werden. Der Gassensor 206 ist ein infrarotoptischer Gassensor, durch den die Konzentration von entlang des zweiten Gasleitungsweges geleitetem gasförmigen Methan messbar ist. Der Leitfähigkeitsdetektor 207 ist ein thermischer Leitfähigkeitsdetektor, durch den die Konzentration von entlang des zweiten Gasleitungsweges geleitetem gasförmigen Wasserstoff messbar ist.

Eine dritte Gasleitung 300 verläuft von einem Abschnitt der ersten Gasleitung 100 zwischen dem zweiten Ende 102 und dem Ventil 104 zu einem Abschnitt der zweiten Gasleitung 200 zwischen dem Ventil 203 und der Gaswaschflasche 204. Durch die dritte Gasleitung 300 ist ein dritter Gasleitungsweg definiert, durch den Gas entlang der dritten Gasleitung 300 von dem vorstehenden Abschnitt der ersten Gasleitung zu dem vorstehenden Abschnitt der zweiten Gasleitung 200 leitbar ist. Die dritte Gasleitung 300 ist durch ein Ventil 301 absperrbar.

Auf dem Leitungsabschnitt der ersten Gasleitung 100 zwischen dem Stickstofftank 103 und dem Abzweig der ersten Gasleitung 100 in die dritte Gasleitung 300 weist die erste Gasleitung 100 einen Durchflussmesser 105 zur Messung des Gasvolumens von durch die erste Gasleitung 100 strömendem Stickstoffgases auf.

Durch den Deckel 6 des Autoklavs 2 ist eine vierte Leitung 400 geführt, die sich von einem ersten Ende 401, an dem die vierte Leitung 400 in den Raum 3 mündet, zu einem zweiten Ende 402 erstreckt. An ihrem zweiten Ende 402 ist die vierte Leitung an einen Wasser enthaltenden Wassertank 403 angeschlossen. Durch die vierte Leitung 400 ist ein Leitungsweg definiert, durch den Wasser entlang der vierten Leitung 400 vom zweiten Ende 402 zum ersten Ende 401 leitbar ist. Die vierte Leitung 400 ist über ein Ventil 404 absperrbar.

### Ausführungsbeispiel 1 für das Verfahren

In der praktischen Anwendung wird gemäß einem ersten Ausführungsbeispiel das erfindungsgemäße Verfahren auf der Vorrichtung 1 wie folgt durchgeführt, wobei die quantitative Bestimmung des gebildeten wenigstens einen Gases, im Ausführungsbeispiel Methan, mittels einer gasanalytischen quantitativen Bestimmung des Methans erfolgt.

Die vorbezeichnete Vorrichtung 1 wird zur Verfügung gestellt. Durch den Autoklav 2 ist damit ein gasdicht verschließbarer Raum 3 zur Verfügung gestellt.

Ferner ist durch den Wassertank 403 eine wässrige Flüssigkeit in Form von Wasser zur Verfügung gestellt.

Um eine Al₄C₃ umfassende Substanz zur Verfügung zu stellen, wurde zunächst aus 88,5 Masse-% MgO, 8 Masse-% C, 2,5 Masse-% Phenolharzbinder und 1 Masse-% Al ein Magnesiakohlenstoffstein hergestellt. Um einen gebrauchten Zustand dieses Magnesiakohlenstoffsteines zu simulieren, wurde der Magnesiakohlenstoffstein für 6 Stunden bei 1.000°C in reduzierender Atmosphäre verkokt, wobei sich aus Teilen des Al und des C des Magnesiakohlenstoffsteines Anteile an Al₄C₃ bildeten. Teile des Al im Magnesiakohlenstoffstein reagierten während des Verkokens mit Stickstoff aus der Luft ferner zu AlN (Aluminiumnitrid). Der verkokte Magnesiakohlenstoffstein wurde auf eine Korngröße unter 1 mm zerkleinert. In dieser Form wurde der entsprechend gebrauchte, zu Schüttgut zerkleinerte Magnesiakohlenstoffstein als Al₄C₃ umfassende Substanz zur Verfügung gestellt.

Bei abgehobenem Deckel 6 wurden 10 g des zu Schüttgut zerkleinerten Magnesiakohlenstoffsteins in den Raum 3 des Autoklavs 2 eingegeben und der Raum 3 anschließend durch den Deckel 6 verschlossen. Ventil 404 blieb dabei noch geschlossen.

Anschließend wurden die Ventile 104 und 203 geöffnet und gasförmiger Stickstoff aus dem Stickstofftank 103 über die erste Gasleitung 100 in den Raum 3 eingeleitet, wobei die im Raum 3 vorhandene Luft aus dem Raum 3 verdrängt wurde und über die zweite Gasleitung 200 aus dem Raum 3 entwich. Anschließend wurden die Ventile 104 und 203 wieder geschlossen.

Bei geöffnetem Ventil 404 wurde über die vierte Leitung 400 anschließend 70 ml Wasser vom Wassertank 403 in dem Raum 3 eingeleitet und der Raum 3 durch Schließen des Ventils 404 anschließend gasdicht verschlossen. Anschließend wurde der Rührer 4 aktiviert, so dass der im Raum 3 befindliche, zerkleinerte Magnesiakohlenstoffstein und das im Raum 3 befindliche Wasser innig miteinander gemischt wurden.

Gleichzeitig wurde die Heizvorrichtung 5 aktiviert und der Raum 3 innerhalb eines Zeitraumes von 30 Minuten gleichmäßig von Raumtemperatur auf eine Temperatur von 150°C aufgeheizt und für einen Zeitraum von 5 Minuten auf dieser Temperatur gehalten. Nach der Haltezeit von 5 Minuten bei 150°C wurde die Heizvorrichtung 5 deaktiviert und der Autoklav 2 von außen mit Wasser gekühlt, worauf die Temperatur im Raum 3 wieder sank.

Während dieser Temperaturbeaufschlagung des Raums 3 und der Vermischung des zerkleinerten Magnesiakohlenstoffsteins mit dem Wasser im Raum 3 reagierte das Al₄C₃ des Magnesiakohlenstoffsteins mit dem Wasser gemäß der folgenden Reaktionsgleichung (I):

Al₄C₃ + 12 H₂O → 4 Al(OH)₃ + 3 CH₄ ^{↑} (I)

Ferner reagierten das AlN des Magnesiakohlenstoffsteins und während des Verkokens nicht reagierte Reste des Al mit dem Wasser gemäß der folgenden Reaktionsgleichungen (II) und (III):

AlN + 3 H₂O → Al(OH)₃ + NH₃ ^{↑} (II)

2 Al + 6 H₂O → 2 Al(OH)₃ + 3 H₂ ^{↑} (III)

Aufgrund der Temperaturbeaufschlagung des Raums 3 und der gemäß den vorstehenden Reaktionsgleichungen (I) bis (III) entstandenen Gase Methan (CH₄), Ammoniak (NH₃) und Wasserstoff (H₂), des in den Raum 3 eingeleiteten Stickstoffgases sowie des entstandenen Wasserdampfs stieg der Druck in dem Raum 3 auf ca. 4 bar Überdruck an, während der Magnesiakohlenstoffstein und das Wasser miteinander reagierten.

Vor dem anschließenden Öffnen des Ventils 203 wurde zunächst noch Ventil 301 geöffnet und gasförmiger Stickstoff aus dem Stickstofftank 103 über die erste Gasleitung 100 und die dritte Gasleitung 300 in die zweite Gasleitung 200 geleitet, um den Gassensor 206 und den Leitfähigkeitsdetektor 207 zu kalibrieren. Zur Unterstützung dieser Leitung des Stickstoffgases wurde die Gasaufbereitungspumpe 205 aktiviert.

Nachdem die Temperatur im Raum 3 auf 45°C gefallen war, wurde Ventil 203 geöffnet, um die im Raum 3 befindlichen Gase (Methan, Ammoniak, Wasserstoff, Wasserdampf und Stickstoff) entlang des durch die zweite Gasleitung 200 definierten zweiten Gasleitungsweges fördern zu können. Zur Förderung dieser im Raum 3 befindlichen Gase entlang des durch die zweite Gasleitung 200 definierten zweiten Gasleitungsweges, wurde Ventil 104 geöffnet und Stickstoff aus dem Stickstofftank 103 über die erste Gasleitung 100 in den Raum 3 geleitet, wobei der Stickstoff die im Raum 3 befindlichen Gase erfasste, über das erste Ende 201 der zweiten Gasleitung 200 den Raum verließ und als Trägergas die Gase anschließend entlang des zweiten Gasleitungsweges förderte. Das Gasvolumen des dabei geförderten Gases wurde mittels des Durchflussmessers 105 bestimmt. Zwar strömt bereits bei Öffnung des Ventils 203 ein Anteil des in dem Raum 3 befindlichen Gases in den zweiten Gasleitungsweg; allerdings ist das Volumen dieses Gasanteils im Verhältnis zu dem durch das Trägergas geförderten Gesamtvolumen vernachlässigbar, so dass das Gasvolumen zuverlässig durch den Durchflussmesser 105 bestimmbar ist.

Bei Förderung der Gase durch das Bad aus 10%-iger Schwefelsäure der Gaswaschflasche 204 wurde zunächst der Ammoniak ausgewaschen.

Ferner wurden die Gase bei deren anschließender Förderung durch die Gasaufbereitungspumpe 205 auf 5°C gekühlt, wodurch Wasserdampf auskondensierte.

Die verbliebenen Gase Methan, Wasserstoff und Stickstoff wurden am Gassensor 206 entlang gefördert, wobei die Konzentration des Methans im Gas durch den Gassensor 206 laufend bestimmt wurde.

Anschließend wurden die verbliebenen Gase am Leitfähigkeitsdetektor 207 entlang gefördert, wobei die Konzentration des Wasserstoffs im Gas durch den Leitfähigkeitsdetektor 207 laufend bestimmt wurde.

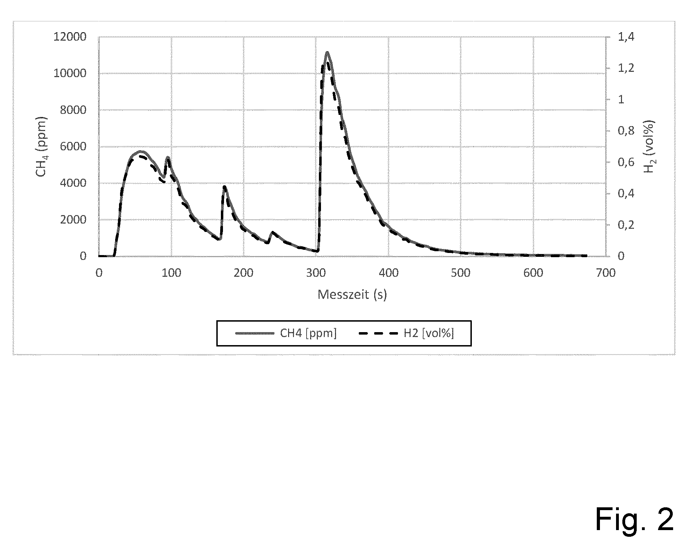

In Figur 2 sind die dabei ermittelten Messergebnisse der vom Gassensors 206 bestimmten Konzentration des Methans in ppm und der vom Leitfähigkeitsdetektor 207 bestimmten Konzentration des Wasserstoffs in Volumen-%, jeweils über die Messzeit in Sekunden, angegeben.

Schließlich wurden die Gase durch den Gasabzug 208 nach außen abgeleitet.

Von den bei der Reaktion des Magnesiakohlenstoffsteines und dem Wasser gebildeten Gasen wurde Methan gasanalytisch quantitativ bestimmt, um auf Basis dieser quantitativen Bestimmung von Methan anschließend die Menge an Al₄C₃ im Magnesiakohlenstoffsteine quantitativ zu bestimmen.

Zunächst wurde gemäß der Gleichung $V \left({}_{CH 4}\right) \left[l\right] = {CH}_{4} \left[ppm\right] * {10}^{- 6} * {N}_{2} \left[l/min\right] * 1 \left[sec\right]$ das Gesamtvolumen des geförderten Methans bestimmt.

Aus den Messergebnissen für die Konzentration des Methans gemäß Figur 2 wurde dabei zunächst die Konzentration des Methans über die gesamte Messdauer bestimmt, was dem Integral über die Zeit ∫ CH₄ dt entspricht. Danach wurde die Summe der Konzentration des Methans über die gesamte Messdauer bestimmt als CH₄ [ppm] = 1,312*10⁶ ppm.

Aus dem am Durchflussmesser 105 eingestellten, gemittelten Volumenstrom über die Messdauer von 1,00/60 [l/sec] wurde dann das Gesamtvolumen des geförderten Methans wie folgt berechnet: $V \left({}_{CH 4}\right) \left[l\right] = 1 , 312 * {10}^{6} ppm * {10}^{- 6} * 1 , 00 / 60 \left[l/sec\right] * 1 \left[sec\right] = 0 , 022 \left[l\right]$

Um die Stoffmenge (in Mol) des Methans zu erhalten, muss das berechnete Volumen durch das Molvolumen des Methans unter Standardbedingungen dividiert werden. Das Molvolumen unter Standardbedingungen errechnete sich gemäß der allgemeinen Gasgleichung ${V}_{mol} \left[1/mol\right] = \left(R*T\right) / p$ mit $R = 8 , 314 \left[\left({kg}^{∗}{m}^{2}\right)/\left({s}^{2 ∗}{mol}^{∗}K\right)\right]$ $T = 273 , 15 \left[K\right]$ $p = 101325 \left[Pa\right] = 101325 \left[kg/\left({m * s}^{2}\right)\right]$ wie folgt: 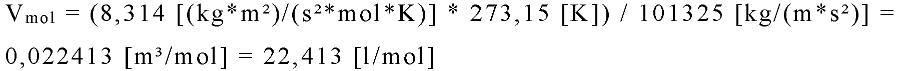 Vmol = (8,314 [(kg*m2)/(s2*mol*K)] * 273,15 [K]) / 101325 [kg/(m*s2)] = 0,022413 [m3/mol] = 22,413 [l/mol]

Die quantitative Bestimmung der Stoffmenge an Methan wurde schließlich gemäß der Gleichung $n {CH}_{4} \left[mol\right] = {V}_{\left(CH4\right)} \left[l\right] / {V}_{mol} \left[l/mol\right]$ wie folgt berechnet: $n {CH}_{4} \left[mol\right] = 0 , 022 \left[l\right] / 22 , 413 \left[l/mol\right] = 9 , 8 * {10}^{- 4} \left[mol\right]$

Für die anschließende quantitative Bestimmung der Stoffmenge an Al₄C₃ im Magnesiakohlenstoffstein wurde anschließend auf Basis dieser Stoffmengenbestimmung für Methan gemäß der Reaktionsgleichung (I):

Al₄C₃ + 12 H₂O → 4 Al(OH)₃ + 3 CH₄ ^{↑} (I),

wonach 1 Mol Al₄C₃ zu 3 Mol CH₄ reagiert (Reaktionsverhältnis 1/3), stöchiometrisch gemäß der folgenden Gleichung zurückgerechnet: ${Al}_{4} {C}_{3} \left[g\right] = n {CH}_{4} \left[mol\right] * 1 / 3 * {M}_{\left(Al4C3\right)} \left[g/mol\right]$ mit $M \left({}_{Al 4 C 3}\right) = Molmasse {Al}_{4} {C}_{3} \left[g/mol\right]$

Damit ließ sich die Stoffmenge an Al₄C₃ im Magnesiakohlenstoffstein wie folgt quantitativ bestimmen: ${Al}_{4} {C}_{3} \left[g\right] = 9 , 8 * {10}^{- 4} \left[mol\right] * 1 / 3 * 143 , 96 \left[g/mol\right] = 0 , 0471 g$

Die Menge an Al₄C₃ im Magnesiakohlenstoffstein wurde damit quantitativ bestimmt mit 0,0471 g.

In Hinblick auf die Probenmenge von 10 g entsprach dies einer Konzentration von Al₄C₃ in dem probengemäßen Magnesiakohlenstoffstein von 4710 ppm.

### Ausführungsbeispiel 2 für das Verfahren

Gemäß einem zweiten Ausführungsbeispiel wurde das erfindungsgemäße Verfahren auf der Vorrichtung 1 wie folgt durchgeführt, wobei die quantitative Bestimmung des gebildeten wenigstens einen Gases, im Ausführungsbeispiel Methan, mittels einer Druckmessung erfolgte.

Das Verfahren gemäß dem zweiten Ausführungsbeispiel wurde im Wesentlichen gemäß dem Verfahren gemäß Ausführungsbeispiel 1 durchgeführt. Allerdings wurde bei der Durchführung des Verfahrens während des Aufheizens des Raums 3 im Temperaturintervall von 50 bis 85°C und während des Abkühlens des Raums 3 im Temperaturintervall von 89 bis 50°C der Überdruck mittels des Drucksensors 7 gemessen. Ferner wurde das Verfahren zweimal durchgeführt, und zwar einmal mit der Probe gemäß Ausführungsbeispiel 1 und ein weiteres Mal mit einem Magnesiakohlenstoffstein ("Nullprobe"), der sich allein insoweit vom Magnesiakohlenstoffstein gemäß Ausführungsbeispiel 1 unterschied, als diesem kein Al zugegeben worden war, so dass sich bei dessen Verkokung kein Al₄C₃ bilden konnte.

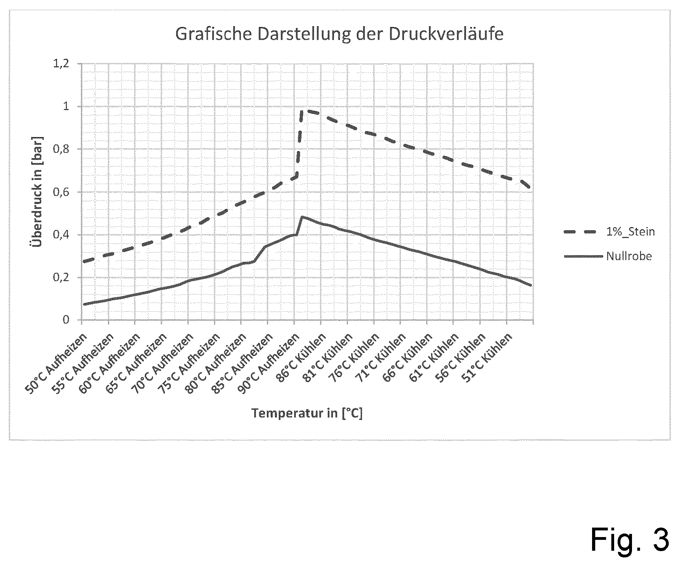

In Figur 3 sind die Messergebnisse für diese Druckmessungen dargestellt. Die durchgezogene Linie zeigt die Messergebnisse für die Druckmessung der Nullprobe, während die gestrichelte Linie die Messergebnisse für die Druckmessung des Magnesiakohlenstoffsteins gemäß Ausführungsbeispiel 1 zeigt.

Von den bei der Reaktion des Magnesiakohlenstoffsteines und dem Wasser gebildeten Gasen wurde Methan mittels der Druckmessung quantitativ bestimmt, um auf Basis dieser quantitativen Bestimmung von Methan anschließend die Menge an Al₄C₃ im Magnesiakohlenstoffsteine quantitativ zu bestimmen.

Dabei wurde zunächst der Überdruck pÜ mit dem Drucksensor 7 (relative Druckmessung) bei 50°C beim Aufheizen und beim Abkühlen gemessen und wie folgt bestimmt: ${p}_{Ü \left(Aufheizen 50°C\right)} = 0 , 2745 \left[bar\right]$ ${p}_{Ü \left(Abkühlen 50°C\right)} = 0 , 65 \left[bar\right]$

Bei Berücksichtigung eines Korrekturfaktors zur Berücksichtigung der Expansion der Luft ergibt sich: 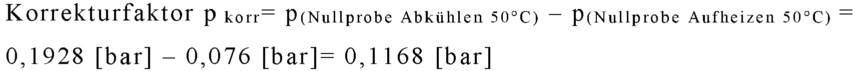 Korrekturfaktor p korr= p(Nullprobe Abkühlen 50°C) - p(Nullprobe Aufheizen 50°C) = 0,1928 [bar] - 0,076 [bar]= 0,1168 [bar] und damit 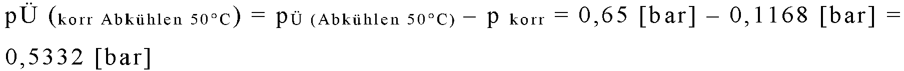 pÜ (korr Abkühlen 50°C) = pÜ (Abkühlen 50°C) - p korr = 0,65 [bar] - 0,1168 [bar] = 0,5332 [bar]

Anschließend wurde der Absolutdruck p [bar] berechnet gemäß: $p \left[bar\right] = {p}_{Ü Korr} \left[bar\right] + 1 , 013 \left[bar\right]$ der somit beim Aufheizen 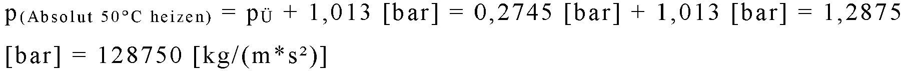 p(Absolut 50°C heizen) = pÜ + 1,013 [bar] = 0,2745 [bar] + 1,013 [bar] = 1,2875 [bar] = 128750 [kg/(m*s2)] und beim Abkühlen 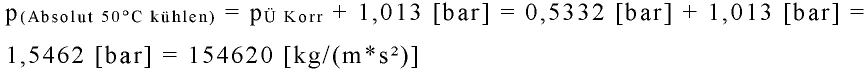 p(Absolut 50°C kühlen) = pÜ Korr + 1,013 [bar] = 0,5332 [bar] + 1,013 [bar] = 1,5462 [bar] = 154620 [kg/(m*s2)] betrug.

Durch die Messung der Wasserstoffkonzentration mittels des Leitfähigkeitsdetektors 207 (Messergebnisse siehe Figur 2) konnte die Reaktionskinetik bestimmt werden. Dies erlaubt die Zuordnung des Verlaufs des Drucksignals zu den Reaktionsprodukten H₂ und CH₄. Das bedeutet, dass die Druckveränderung beim Aufheizen hauptsächlich dem H₂ und beim Abkühlen hauptsächlich dem CH₄ geschuldet ist.

Gemäß der allgemeinen Gasgleichung $p * V / \left(R*T\right) = n$ mit $Freies Volumen V \left[1\right] = 0 , 1 \left[1\right] = 0 , 0001 \left[{m}^{3}\right]$ $Dichte Luft {ρ}_{Luft} \left[kg/{m}^{3}\right] = 1 , 1877 \left[kg/{m}^{3}\right] = 1 , 1877 \left[g/l\right]$ $Molare Masse Luft {M}_{Luft} \left[g/mol\right] = 28 , 949 \left[g/mol\right]$ $R = 8 , 314 \left[\left({kg}^{∗}{m}^{2}\right)/\left({s}^{2 ∗}{mol}^{∗}K\right)\right]$ ${T}_{50 ° C} = 273 , 15 \left[K\right] + 50 \left[K\right] = 323 , 15 \left[K\right]$ mit der Masse der Luft ${\text{Masse Luft m}}_{\text{Luft}} \left[\text{g}\right] = \text{V¨} \left[1\right] * {\text{ρ}}_{\text{Luft}} = 0,1 \left[1\right] * 1,1877 \left[\text{g/l}\right] = 0,11877 \left[\text{g}\right]$ und damit den Mol an Luft ${n}_{Luft} \left[mol\right] = {m}_{Luft} / {M}_{Luft} = 0 , 11877 \left[g\right] / 28 , 949 \left[g/mol\right] = 0 , 0041027 \left[mol\right]$ ergibt sich für die Mol an Wasserstoff: ${n}_{H 2 \left(Aufheizen 50°C\right)} = {p}_{Absolut \left(50°C Aufheizen\right)} ∗ V / \left(R*T\right) - {n}_{Luft}$ 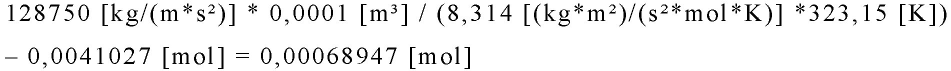 128750 [kg/(m*s2)] * 0,0001 [m3] / (8,314 [(kg*m2)/(s2*mol*K)] *323,15 [K]) - 0,0041027 [mol] = 0,00068947 [mol]

Damit ließ sich die Stoffmenge an Methan wie folgt quantitativ bestimmen: ${n}_{CH 4 50 ° C} \left[mol\right] = {p}_{\left(Absolut 50°C Abkühlen\right)} * V / \left({R * T}_{50 ° C}\right) - {n}_{Luft} - {n}_{H 2 Aufheizen 50 ° C}$ 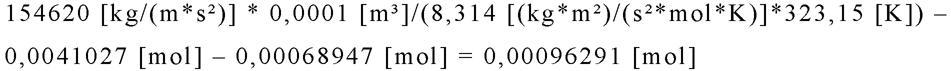 154620 [kg/(m*s2)] * 0,0001 [m3]/(8,314 [(kg*m2)/(s2*mol*K)]*323,15 [K]) - 0,0041027 [mol] - 0,00068947 [mol] = 0,00096291 [mol]

Für die anschließende quantitative Bestimmung der Stoffmenge an Al₄C₃ im Magnesiakohlenstoffstein wurde anschließend auf Basis dieser Stoffmengenbestimmung für Methan gemäß der Reaktionsgleichung (I), wonach 1 Mol Al₄C₃ zu 3 Mol CH₄ reagiert (Reaktionsverhältnis 1/3), stöchiometrisch wie folgt zurückgerechnet: 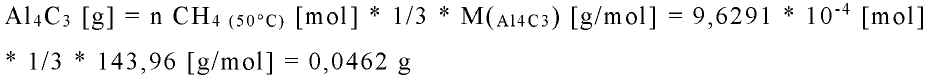 Al4C3 [g] = n CH4 (50°C) [mol] * 1/3 * M(Al4C3) [g/mol] = 9,6291 * 10-4 [mol] * 1/3 * 143,96 [g/mol] = 0,0462 g

Die Menge an Al₄C₃ im Magnesiakohlenstoffstein wurde damit quantitativ bestimmt mit 0,0462 g.

In Hinblick auf die Probenmenge von 10 g entsprach dies einer Konzentration von Al₄C₃ in dem probengemäßen Magnesiakohlenstoffstein von 4620 ppm.

### Bewertung der quantitativen Bestimmung des Al₄C₃ gemäß den Ausführungsbeispielen 1 und 2

Die Ausführungsbeispiele 1 und 2 zeigen, dass das erfindungsgemäße Verfahren eine zuverlässige, einfache und sichere quantitative Bestimmung von Al₄C₃ ermöglicht.

Die Zuverlässigkeit folgt insbesondere auch aus einem Vergleich der Ergebnisse für die Bestimmung gemäß den Ausführungsbeispielen 1 und 2. Danach betrug die relative Abweichung der Messergebnisse für die quantitative Bestimmung an Al₄C₃ nur etwa 1,9 %.

Die Einfachheit des Verfahrens folgt insbesondere auch aus der einfachen Durchführung des Verfahrens, bei dem unter anderem aufgrund der Reaktion in einem gasdicht verschlossenen Raum nicht während der gesamten Reaktionszeit eine quantitative Bestimmung des Methans durchzuführen ist, sowie der einfachen quantitativen Bestimmung von Al₄C₃ mittels Berechnung auf Basis der quantitativen Bestimmung des Methans.

Die Sicherheit des Verfahrens ergibt sich insbesondere auch aus der Verwendung von Wasser für die Reaktion des Al₄C₃.

## Patentansprüche

1. Verfahren zur quantitativen Bestimmung von Al₄C₃, umfassend die folgenden Schritte:
A. Zur Verfügungstellung eines gasdicht verschließbaren Raumes;
B. zur Verfügungstellung einer Al₄C₃ umfassenden Substanz, wobei die Al₄C₃ umfassende Substanz in Form eines feuerfesten Erzeugnisses zur Verfügung gestellt wird;
C. zur Verfügungstellung wenigstens einer wässrigen Flüssigkeit, die unter Bildung wenigstens eines Gases mit Al₄C₃ reagiert;
D. anordnen der Al₄C₃ umfassenden Substanz und der wenigstens einen wässrigen Flüssigkeit in dem Raum;
E. gasdichtes Verschließen des Raumes;
F. reagieren lassen des Al₄C₃ der Al₄C₃ umfassenden Substanz und der wenigstens einen wässrigen Flüssigkeit in dem Raum unter Bildung des wenigstens einen Gases;
G. quantitative Bestimmung des gebildeten wenigstens einen Gases;
H. quantitative Bestimmung von Al₄C₃ in der Al₄C₃ umfassenden Substanz auf Basis der quantitativen Bestimmung des gebildeten wenigstens einen Gases; wobei
I. der Raum während der Reaktion des Al₄C₃ der Al₄C₃ umfassenden Substanz mit der wenigstens einen wässrigen Flüssigkeit in dem Raum unter Überdruck im Bereich von 2-6 bar steht.

2. Verfahren nach Anspruch 1, wobei das wenigstens eine Gas Methan umfasst.

3. Verfahren nach wenigstens einem der vorhergehenden Ansprüche, wobei die wenigstens eine wässrige Flüssigkeit Wasser umfasst, vorzugsweise Wasser darstellt.

4. Verfahren nach wenigstens einem der vorhergehenden Ansprüche, wobei die Al₄C₃ umfassenden Substanz und die wenigstens eine wässrige Flüssigkeit miteinander gemischt werden.

5. Verfahren nach wenigstens einem der vorhergehenden Ansprüche, wobei die Al₄C₃ umfassende Substanz kohlenstoffgebunden ist.

6. Verfahren nach wenigstens einem der vorhergehenden Ansprüche, wobei die Al₄C₃ umfassende Substanz als Schüttgut zur Verfügung gestellt wird.

7. Verfahren nach wenigstens einem der vorhergehenden Ansprüche, wobei der Raum während der Reaktion des Al₄C₃ der Al₄C₃ umfassenden Substanz mit der wenigstens einen wässrigen Flüssigkeit in dem Raum mit Temperatur über Raumtemperatur beaufschlagt wird.

8. Verfahren nach wenigstens einem der vorhergehenden Ansprüche, wobei der Raum durch einen Autoklav (2) zur Verfügung gestellt wird.

9. Verfahren nach wenigstens einem der vorhergehenden Ansprüche, wobei die quantitative Bestimmung des gebildeten wenigstens einen Gases erst nach vollständiger Reaktion des Al₄C₃ der Al₄C₃ umfassenden Substanz und der wenigstens einen wässrigen Flüssigkeit in dem Raum unter Bildung des wenigstens einen Gases erfolgt.

10. Verfahren nach wenigstens einem der vorhergehenden Ansprüche, wobei das wenigstens eine Gas mittels eines infrarotoptischen Gassensors (206) quantitativ bestimmt wird.

11. Verfahren nach wenigstens einem der vorhergehenden Ansprüche, wobei das wenigstens eine Gas mittels eines Drucksensors quantitativ bestimmt wird.

## Claims

1. Method for the quantitative determination of Al₄C₃, comprising the following steps:
A. providing a gas-tight sealable chamber;
B. providing a substance comprising Al₄C₃, wherein the substance comprising Al₄C₃ is provided in the form of a refractory product;
C. providing at least one aqueous liquid that reacts with Al₄C₃ to form at least one gas;
D. placing the substance comprising Al₄C₃ and the at least one aqueous liquid in the chamber;
E. sealing the chamber in a gas-tight manner;
F. reacting the Al₄C₃ of the substance comprising Al₄C₃ and the at least one aqueous liquid in the chamber to form the at least one gas;
G. quantitatively determining the at least one gas formed;
H. quantitatively determining Al₄C₃ in the substance comprising Al₄C₃ based on the quantitative determination of the at least one gas formed; wherein
I. wherein the chamber is under positive pressure in a range of 2-6 bar during the reaction of the Al₄C₃ of the substance comprising Al₄C₃ with the at least one aqueous liquid in the chamber.

2. The method according to claim 1, wherein the at least one gas comprises methane.

3. The method according to at least one of the preceding claims, wherein the at least one aqueous liquid comprises water, preferably is water.

4. The method according to at least one of the preceding claims, wherein the substance comprising Al₄C₃ and the at least one aqueous liquid are mixed together.

5. The method according to at least one of the preceding claims, wherein the substance comprising Al₄C₃ is carbon bonded.

6. The method according to at least one of the preceding claims, wherein the substance comprising Al₄C₃ is provided in bulk.

7. The method according to at least one of the preceding claims, wherein the chamber is subjected to temperature above room temperature during the reaction of the Al₄C₃ of the substance comprising Al₄C₃ with the at least one aqueous liquid in the chamber.

8. The method according to at least one of the preceding claims, wherein the chamber is provided by an autoclave (2).

9. The method according to at least one of the preceding claims, wherein the quantitative determination of the formed at least one gas is performed only after complete reaction of the Al₄C₃ of the substance comprising Al₄C₃ and the at least one aqueous liquid in the chamber to form the at least one gas.

10. The method according to at least one of the preceding claims, wherein the at least one gas is quantitatively determined by means of an infrared optical gas sensor (206).

11. The method according to at least one of the preceding claims, wherein the at least one gas is quantitatively determined by means of a pressure sensor.

## Revendications

1. Procédé de dosage de Al₄C₃, comprenant les étapes suivantes :
A. mise à disposition d'une enceinte pouvant être fermée de manière à être étanche aux gaz ;
B. mise à disposition d'une substance comprenant du AL₄C₃, la substance contenant du AL₄C₃ étant mise à disposition sous forme d'un produit réfractaire ;
C. mise à disposition d'au moins un liquide aqueux qui réagit avec du AL₄C₃ en générant au moins un gaz ;
D. disposer l'au moins une substance contenant du AL₄C₃ et l'au moins un liquide aqueux dans ladite enceinte ;
E. fermer ladite enceinte de manière à ce qu'elle soit étanche aux gaz ;
F. faire réagir le Al₄C₃ de la substance contenant du AL₄C₃ et l'au moins un liquide aqueux dans ladite enceinte en générant l'au moins un gaz ;
G. doser l'au moins un gaz généré ;
H. doser le Al₄C₃ dans la substance comprenant du Al₄C₃ en se basant sur le dosage de l'au moins un gaz généré ; alors que
I. ladite enceinte est placée, pendant la réaction du Al₄C₃ de la substance comprenant du Al₄C₃ avec l'au moins un liquide aqueux, sous une surpression comprise entre 2 et 6 bar.

2. Procédé selon la revendication 1, l'au moins un gaz comprenant du méthane.

3. Procédé selon au moins une des revendications précédentes, l'au moins un liquide aqueux comprenant de l'eau et, préférentiellement, étant constitué d'eau.

4. Procédé selon au moins une des revendications précédentes, la substance comprenant du Al₄C₃ et l'au moins un liquide aqueux étant mélangés entre eux.

5. Procédé selon au moins une des revendications précédentes, la substance comprenant du Al₄C₃ étant liée à du carbone.

6. Procédé selon au moins une des revendications précédentes, la substance contenant du Al₄C₃ étant mise en disposition sous forme d'une matière en vrac.

7. Procédé selon au moins une des revendications précédentes, ladite enceinte étant, pendant la réaction du Al₄C₃ de la substance comprenant du Al₄C₃ avec le liquide aqueux au sein de ladite enceinte, soumise à une température supérieure à la température ambiante.

8. Procédé selon au moins une des revendications précédentes, ladite enceinte étant mise à disposition par un autoclave (2).

9. Procédé selon au moins une des revendications précédentes, le dosage de l'au moins un gaz généré étant réalisé après que la réaction du Al₄C₃ de la substance contenant du Al₄C₃ et de l'au moins un liquide aqueux, au sein de ladite enceinte en générant l'au moins un gaz, est terminée.

10. Procédé selon au moins une des revendications précédentes, l'au moins un gaz étant dosé au moyen d'un capteur optique infrarouge (206) de gaz.

11. Procédé selon au moins une des revendications précédentes, l'au moins un gaz étant dosé au moyen d'un capteur de pression.
